# EUROPÄISCHE PATENTSCHRIFT

(11) **EP 3 359 093 B1**
(45) Veröffentlichungstag und Bekanntmachung des Hinweises auf die Patenterteilung: **11.12.2019**
(21) Anmeldenummer: 16774964.7
(22) Anmeldetag: 04.10.2016
(51) Int. Cl.: A61F 2/38, A61L 27/10, A61F 2/30

(54) **KNIEENDOPROTHESE ZUM ERSATZ VON ZUMINDEST TEILEN DES KNIEGELENKS**
KNEE ENDOPROSTHESIS FOR THE REPLACEMENT OF AT LEAST PART OF THE KNEE JOINT
ENDOPROTHÈSE DE GENOU DESTINÉE À REMPLACER AU MOINS PARTIELLEMENT L'ARTICULATION DU GENOU

(30) Priorität: 07.10.2015 DE 102015219344
(43) Veröffentlichungstag der Anmeldung: 15.08.2018
(73) Patentinhaber: CeramTec GmbH, 73207 Plochingen (DE)
(72) Erfinder: BLÖMER, Wilhelm, 88690 Uhldingen-Mühlhofen (DE)
(86) Internationale Anmeldenummer: PCT/EP2016/073640
(87) Internationale Veröffentlichungsnummer: WO 2017/060221

(56) Entgegenhaltungen:
- US-A- 6 013 103

## Beschreibung

Die Erfindung betrifft eine Knieendoprothese mit einer zwei Kondylenflächen aufweisenden femoralen Komponente zur Verankerung am distalen Femur und mit einer tibialen Komponente zur Verankerung an der proximalen Tibia und mit Gleitflächen zwischen diesen zwei Komponenten nach dem Oberbegriff des Anspruchs 1 oder 2.

Die Erfindung betrifft generell die Verbesserung einer Knieendoprothese zum Ersatz von mindestens Teilen des Kniegelenks. Eine Knieendoprothese für den partiellen bzw. totalen Kniegelenksersatz besteht typischerweise aus einer femoralen Komponente zur Verankerung am distalen Femur, und einer tibialen Komponente zur Verankerung an der proximalen Tibia, wobei die Tibiakomponente ein Insert oder eine Meniskuskomponente zur Artikulation mit der Femurkomponente beinhaltet.

In der gattungsbildenden US 6,013,103 ist eine Knieendoprothese beschrieben, bei der die Oberfläche des Tibiateils eben ausgebildet ist und eine Verschiebung des Meniskusteils in dieser Ebene erlaubt, wobei das Meniskusteil sowohl die mediale als laterale Kondylengleitfläche beinhaltet. Die mediale Kondyle der femoralen Komponente und die mediale Lagerschale des Meniskusteils sind ebenfalls sphärisch ausgebildet. Das Meniskusteil beinhaltet somit drei Gleiflächen -mediale Kondyle, laterale Kondyle und Oberfläche Tibiateil/Unterseite Meniskusteil.

In der Praxis hat sich herausgestellt, dass mit Knieendoprothesen dieser Bauart zwar die anatomischen und kinematischen Verhältnisse relativ gut nachgebildet werden können, bedingt durch die insgesamt drei Reibflächen und die materialtechnisch nicht optimal aufeinander abgestimmten Gleitflächen einen gegenüber fest stehenden Meniskusteilen erhöhten Materialabrieb aufweisen und somit ein erhöhtes Risiko einer Implantatlockerung haben.

Der Erfindung liegt die Aufgabe zugrunde, eine Knieendoprothese so auszubilden, dass sie bei einer optimalen Nachbildung der anatomischen und kinematischen Eigenschaften des natürlichen Kniegelenks, insbesondere durch geeignete Materialkombinationen und eine Reduktion der Abrieb generierenden Flächen einen kleinstmöglichen Abrieb aufweist.

Erfindungsgemäß wird diese Aufgabe durch eine Knieendoprothese nach den Merkmalen des Anspruchs 1 oder 2 gelöst. Dadurch, dass die gesamte femorale Komponente, aber zumindest deren Kondylenflächen, zur Artikulation mit der tibialen Komponente, aus einer fest gesinterten Keramik besteht, dass die tibiale Komponente ein Tibiaplateau aus einer fest gesinterten Keramik umfasst, welches auf seiner zur femolaren Komponente gewandten Oberseite ein mediale sphärische Lagerschale aufweist, wobei die mediale sphärische Lagerschale integraler Bestandteil des Tibiaplateaus und einstückig mit ihm ausgebildet ist und die Artikulationsfläche der medialen Femurkondyle ebenfalls sphärisch ausgebildet ist und zusammen mit der medialen sphärischen Lagerschale ein kongruentes Kugelgelenk darstellt, und lateral neben der medialen sphärischen Lagerschale eine die Rotation der femoralen Komponente ermöglichende, in anterior - posteriorer Richtung aus mehreren Radien gebildete und zur Femurkondyle nicht kongruente Lagerschale angeordnet ist, die entweder einstückig mit dem Tibiaplateau ausgebildet ist oder ein relativ zum Tibiaplateau bewegliches Inlay ist, weisen die eine Reduktion der Abrieb generierenden Flächen einen kleinstmöglichen Abrieb auf.

Bevorzugt weist das Tibiaplateau an seiner zur proximalen Tibia gewandten Unterseite einen Bolzen zur Verankerung auf. In vorteilhafter Weise besteht der Bolzen aus einer fest gesinterten Keramik und ist einstückig mit dem Tibiaplateau ausgebildet. Der Bolzen kann einen nicht kreisförmigen Querschnitt haben und ist damit drehfest in der natürlichen Tibia verankert.

Das Inlay besteht bevorzugt aus UHMWPE, PEEK, PAEK oder Kompositen oder aus einer fest gesinterten Keramik oder Polyethylene.

Das Inlay besteht bevorzugt aus UHMWPE, PEEK, PAEK oder Kompositen oder aus einer fest gesinterten Keramik oder Polyethylene.

Das Inlay kann in einer Nut auf dem Tibiaplateau beweglich sein, wodurch eine Rotation der femoralen Komponente ermöglicht ist. Die Nut ist vorteilhafterweise in anterior-posteriorer Richtung geradlinig oder mit einem die Femurrotation bestimmenden Krümmungsradius ausgebildet.

Die Femurrotation ist bevorzugt auf maximal 15 Grad interner und externer Rotation begrenzt.

Zur Gelenkstabilisierung in Extension (Streckstellung) weist bevorzugt die Lagerschale oder das Inlay in anteriorer Richtung eine Erhöhung aufweist.

Die Kondylenfläche 1b, die in der Lagerschale artikuliert weist vorteilhaft neben der Flexionsbewegung auch eine Translationsbewegung in anterior-posteriorer Richtung auf.

Eine erfindungsgemäße tibiale Komponente einer Knieendopothese ist dadurch gekennzeichnet, dass die tibiale Komponente ein Tibiaplateau aus einer fest gesinterten Keramik umfasst, welches eine mediale sphärische Lagerschale aufweist, die integraler Bestandteil des Tibiaplateaus ist und einstückig mit ihr ausgebildet ist und neben der medialen sphärischen Lagerschale eine die Rotation der femoralen Komponente ermöglichende, in anterior - posteriorer Richtung aus mehreren Radien gebildete und zur Femurkondyle nicht kongruente Lagerschale angeordnet ist, die entweder einstückig mit dem Tibiaplateau ausgebildet ist oder ein relativ zum Tibiaplateau bewegliches Inlay ist.

Die Erfindung betrifft somit eine Knieendoprothese mit einem Tibiateil, mit einem zwei Kondylenflächen aufweisenden Femurteil und nur einem ausschließlich zwischen lateraler Femurkondyle und Tibiateil angeordneten Meniskusteils, welches an seiner Oberseite eine Lagerschale zur Aufnahme und Lagerung der lateralen Kondylenflächen des Femurteils und welches an seiner Unterseite einen Verankerungsmechanismus zur festen aber lösbaren Verbindung mit dem Tibiateil aufweist, wobei die mediale Lagerschale integraler Bestandteil des Tibiaplateaus ist und die Lagerschale eine sphärische Geometrie aufweist und zusammen mit der sphärischen Geometrie der medialen Femurkondyle ein kongruentes Kugelgelenk darstellt, wobei das Tibiateil mit der medialen Lagerschale ein gemeinsames Bauteil (Monoblock) bildet und vorzugsweise aus einem keramischen Material hergestellt ist und mit dem ebenfalls aus Keramik hergestellten Femurteil artikuliert. Das modulare laterale Meniskusteil -fixed oder mobile- ist vorzugsweise aus UHMWPE hergestellt und artikuliert ebenfalls mit der keramischen Femurkondyle.

Dadurch, dass die mediale Lagerschale integraler Bestandteil des keramischen Tibiaplateaus und sphärisch ausgebildet ist, bietet dieses mediale Kugelgelenk eine größtmögliche Kongruenz durch eine Keramik/ Keramik Gleitpaarung vergleichbar zur Keramik/Keramik Artikulation in der Hüftendeprothetik und verspricht einen minimalen Abrieb. Lateral gewährleistet eine die Rotation der Femurkomponente ermöglichende, zur Femurkondyle nicht kongruente Lagerschale eines mit dem keramischen Tibiateil zu verbindenden oder relativ zum Tibiateil beweglichen Inlays eine weitgehend mit dem natürlichen Gelenk vergleichbare Kinematik, wobei das Inlay aus UHMWPE, PEEK, PAEK, Kompositen aus verschiedenen Materialien wie z.B Keramik/ Polyethylene hergestellt ist.

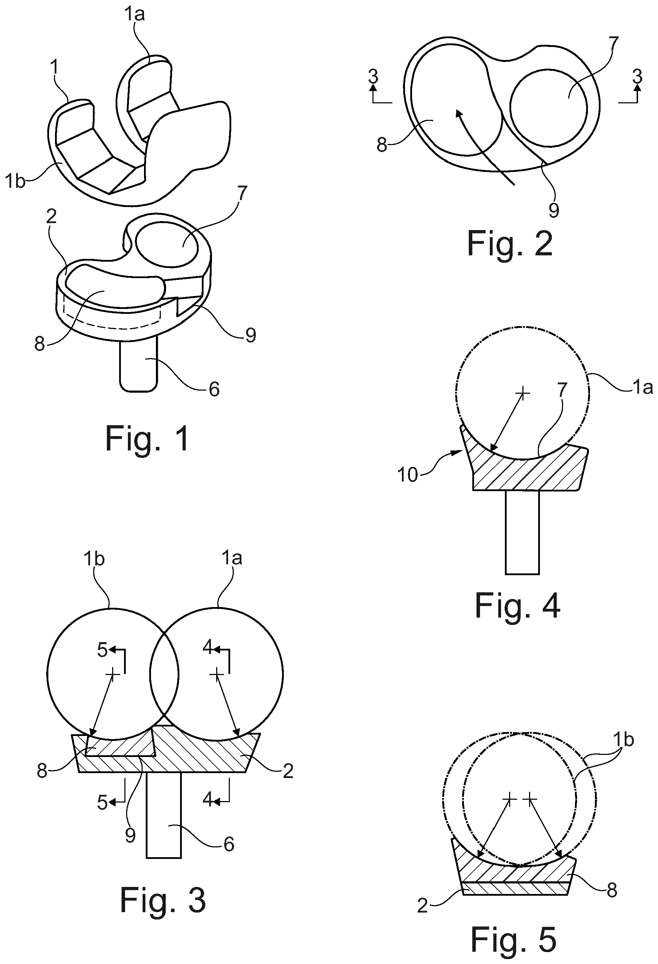

Figur 1 zeigt eine erfindungsgemäße Kniegelenkendoprothese mit einem eine femorale Komponente 1 aufweisenden Femurteil, welches aus zwei Kondylenflächen 1a, 1b besteht, zur Verankerung am distalen Femur und einer tibialen Komponente 2 zur Verankerung an der proximalen Tibia. Die tibiale Komponente besteht aus einem Tibiaplateau, an dessen zur Tibia gewandten Unterseite zur Verankerung ein Bolzen 6 angeordnet ist. Auf der Oberseite, d.h. auf der zur femoralen Komponente 1 gewandten Seite des Tibiaplateau ist erfindungsgemäß eine mediale Lagerschale 7 als integraler Bestandteil des keramischen Tibiaplateaus angeordnet. Diese mediale Lagerschale 7 ist sphärisch ausgebildet. Sowohl die femorale Komponente 1 als auch die tibiale Komponente 2 sind aus einer fest gesinterten Keramik gefertigt worden, d.h. sind Keramikteile. Hierdurch ist eine Keramik/Keramik Gleitpaarung geschaffen, die nahezu keinen Abrieb auch bei langem Gebrauch aufweist. Lateral ist neben der medialen Lagerschale 7 eine die Rotation der Femurkomponente ermöglichende, in anterior - posteriorer Richtung aus mehreren Radien gebildete und somit zur Femurkondyle nicht kongruente Lagerschale 8 angeordnet. Diese Lagerschale 8 kann ein mit der tibialen Komponente 2 fest verbundene Lagerschale oder ein relativ zur tibialen Komponente 2 bewegliches Inlay sein, welches eine weitgehend mit dem natürlichen Gelenk vergleichbare Kinematik aufweist. Das bewegliche Inlay besteht bevorzugt aus UHMWPE, PEEK, PAEK oder Kompositen oder aus Materialien wie Keramik oder Polyethylene.
Figur 1 zeigt eine Ausführungsform einer Lagerschale 8, die in einer Nut 9 in der tibialen Komponente 2 beweglich ist. Diese Nut 9 kann in anterior-posteriorer Richtung geradlinig oder auch mit einem die Femurrotation bestimmenden Krümmungsradius ausgebildet sein, wobei die Femurrotation auf ca. 15 Grad interner und externer Rotation begrenzt wird (nicht dargestellt). Der Krümmungsradius variiert in Abhängigkeit der jeweiligen Größe der Femurkomponente.
Figur 2 zeigt die tibiale Komponente 2 gemäß Figur 1 als Draufsicht auf das zur femoralen Komponente 1 gewandte Tibiaplateau. Gut zu erkennen ist die mediale Lagerschale 7, die sphärisch ausgebildet ist. Neben der medialen Lagerschale 7 ist die zur Femurkondyle nicht kongruente Lagerschale 8 angeordnet, die sowohl fest mit dem Tibiaplateau verbunden sein kann (nicht dargestellt) oder beweglich in der Nut 9 auf dem Tibiaplateau geführt ist.
Figur 3 zeigt einen Schnitt entlang der Linie 3-3 in der Figur 2. Die zur Femurkondyle 1b in anterior-posteriorer Richtung nicht kongruente Lagerschale 8 ist auf dem Tibiaplateau fest verankert oder in einer Nut 9 beweglich eingesetzt, während die Kondylenfläche 1a kongruent in der sphärischen medialen Lagerschale 7 artikuliert.
Figur 4 zeigt einen Schnitt entlang der Linie 4-4 in der Figur 3. Die Kondylenfläche 1a artikuliert kongruent in der sphärischen medialen Lagerschale 7. Zur Gelenkstabilisierung in Extension (Streckstellung), weist die Lagerschale 7 in anteriorer Richtung eine Erhöhung 10 auf.
Figur 5 zeigt einen Schnitt entlang der Linie 5-5 in der Figur 3. Die Kondylenfläche 1b artikuliert in der Lagerschale 8 und ermöglicht neben der Flexionsbewegung auch eine Translationsbewegung in anterior-posteriorer Richtung. Figur 5 zeigt zwei mögliche Stellungen sowie den Translationsweg der Kondylenfläche 1b.

### Bezugszeichen:

- 1.: femorale Komponente, 1a Kondylenflächen
- 2.: tibiale Komponente
- 3.: Linie 3-3
- 4.: Linie 4-4
- 5.: Linie 5-5
- 6.: Bolzen
- 7.: mediale Lagerschale
- 8.: kongruente Lagerschale
- 9.: Nut
- 10.: Erhöhung

## Patentansprüche

1. Knieendoprothese mit einer zwei Kondylenflächen (1a, 1b) aufweisenden femoralen Komponente (1) zur Verankerung am distalen Femur und mit einer tibialen Komponente (2) zur Verankerung an der proximalen Tibia und mit Gleitflächen zwischen diesen zwei Komponenten (1, 2), wobei das Tibiaplateau auf seiner zur femolaren Komponente (1) gewandten Oberseite eine mediale sphärische Lagerschale (7) aufweist, wobei die mediale sphärische Lagerschale (7) integraler Bestandteil des Tibiaplateaus und einstückig mit ihm ausgebildet ist und die Artikulationsfläche der medialen Femurkondyle (1a) ebenfalls sphärisch ausgebildet ist und zusammen mit der medialen sphärischen Lagerschale (7) ein kongruentes Kugelgelenk darstellt, und lateral neben der medialen sphärischen Lagerschale (7) eine die Rotation der femoralen Komponente (1) ermöglichende, in anterior - posteriorer Richtung aus mehreren Radien gebildete und zur Femurkondyle (1b) nicht kongruente Lagerschale (8) angeordnet ist, die einstückig mit dem Tibiaplateau ausgebildet ist, **dadurch gekennzeichnet, dass** die gesamte femorale Komponente (1), aber zumindest deren Kondylenflächen (1a, 1b), zur Artikulation mit der tibialen Komponente (2), aus einer fest gesinterten Keramik besteht, dass die tibiale Komponente (2) ein Tibiaplateau aus einer fest gesinterten Keramik umfasst.

2. Knieendoprothese mit einer zwei Kondylenflächen (1a, 1b) aufweisenden femoralen Komponente (1) zur Verankerung am distalen Femur und mit einer tibialen Komponente (2) zur Verankerung an der proximalen Tibia und mit Gleitflächen zwischen diesen zwei Komponenten (1, 2), wobei das Tibiaplateau auf seiner zur femolaren Komponente (1) gewandten Oberseite eine mediale sphärische Lagerschale (7) aufweist, wobei die mediale sphärische Lagerschale (7) integraler Bestandteil des Tibiaplateaus und einstückig mit ihm ausgebildet ist und die Artikulationsfläche der medialen Femurkondyle (1a) ebenfalls sphärisch ausgebildet ist und zusammen mit der medialen sphärischen Lagerschale (7) ein kongruentes Kugelgelenk darstellt, und lateral neben der medialen sphärischen Lagerschale (7) eine die Rotation der femoralen Komponente (1) ermöglichende, in anterior - posteriorer Richtung aus mehreren Radien gebildete und zur Femurkondyle (1b) nicht kongruente Lagerschale (8) angeordnet ist, die ein relativ zum Tibiaplateau bewegliches Inlay ist, **dadurch gekennzeichnet, dass** die gesamte femorale Komponente (1), aber zumindest deren Kondylenflächen (1a, 1b), zur Artikulation mit der tibialen Komponente (2), aus einer fest gesinterten Keramik besteht, dass die tibiale Komponente (2) ein Tibiaplateau aus einer fest gesinterten Keramik umfasst.

3. Knieendoprothese nach Anspruch 1, **dadurch gekennzeichnet, dass** das Tibiaplateau an seiner zur proximalen Tibia gewandten Unterseite einen Bolzen (6) zur Verankerung aufweist.

4. Knieendoprothese nach Anspruch 2, **dadurch gekennzeichnet, dass** das Tibiaplateau an seiner zur proximalen Tibia gewandten Unterseite einen Bolzen (6) zur Verankerung aufweist.

5. Knieendoprothese nach Anspruch 2 oder 4, **dadurch gekennzeichnet, dass** das Inlay aus UHMWPE, PEEK, PAEK oder Kompositen oder aus einer fest gesinterten Keramik oder Polyethylene besteht.

6. Knieendoprothese nach einem der Ansprüche 2, 4 oder 5, **dadurch gekennzeichnet, dass** das Inlay in einer Nut (9) auf dem Tibiaplateau beweglich ist.

7. Knieendoprothese nach Anspruch 6, **dadurch gekennzeichnet, dass** die Nut (9) in anterior-posteriorer Richtung geradlinig oder mit einem die Femurrotation bestimmenden Krümmungsradius ausgebildet ist.

8. Knieendoprothese nach Anspruch 7, **dadurch gekennzeichnet, dass** die Femurrotation auf maximal 15 Grad interner und externer Rotation begrenzt ist.

9. Knieendoprothese nach Anspruch 1 oder 3, **dadurch gekennzeichnet, dass** die Lagerschale (8) in anteriorer Richtung eine Erhöhung (10) aufweist.

10. Knieendoprothese nach einem der Ansprüche 2, 4 bis 8, **dadurch gekennzeichnet, dass** das Inlay in anteriorer Richtung eine Erhöhung (10) aufweist.

11. Knieendoprothese nach einem der Ansprüche 1 bis 10, **dadurch gekennzeichnet, dass** die Kondylenfläche (1b) in der Lagerschale (8) artikuliert und neben der Flexionsbewegung auch eine Translationsbewegung in anterior-posteriorer Richtung aufweisen kann.

12. Tibiale Komponente einer Knieendoprothese mit einer zwei Kondylenflächen (1a, 1b) aufweisenden femoralen Komponente (1) zur Verankerung am distalen Femur und mit einer tibialen Komponente (2) zur Verankerung an der proximalen Tibia und mit Gleitflächen zwischen diesen zwei Komponenten (1, 2) nach einem der Ansprüche 1 bis 11, wobei die tibiale Komponente (2) ein Tibiaplateau aus einer fest gesinterten Keramik umfasst, welches auf seiner zur femolaren Komponente (1) gewandten Oberseite eine mediale sphärische Lagerschale (7) aufweist, die integraler Bestandteil des Tibiaplateaus ist und einstückig mit ihr ausgebildet ist und die Artikulationsfläche der medialen Femurkondyle (1a) ebenfalls sphärisch ausgebildet ist und zusammen mit der medialen sphärischen Lagerschale (7) ein kongruentes Kugelgelenk darstellt, und lateral neben der medialen sphärischen Lagerschale (7) eine die Rotation der femoralen Komponente (1) ermöglichende, in anterior - posteriorer Richtung aus mehreren Radien gebildete und zur Femurkondyle nicht kongruente Lagerschale (8) angeordnet ist, die entweder einstückig mit dem Tibiaplateau ausgebildet ist oder ein relativ zum Tibiaplateau bewegliches Inlay ist.

## Claims

1. Knee endoprosthesis comprising a femoral component (1), which has two condylar surfaces (1a, 1b), for anchoring to the distal femur, and comprising a tibial component (2) for anchoring to the proximal tibia, and comprising sliding surfaces between said two components (1, 2), the tibial plateau having a medial spherical bearing shell (7) on the upper face of said plateau that faces the femoral component (1), the medial spherical bearing shell (7) being an integral component of the tibial plateau and integrally formed therewith, and the articulation surface of the medial femoral condyle (1a) also being spherical and, together with the medial spherical bearing shell (7), constituting a congruent ball joint, and a bearing shell (8) being arranged laterally adjacent to the medial spherical bearing shell (7), which laterally arranged shell allows the rotation of the femoral component (1), is formed in the anterior-posterior direction from a plurality of radii, is not congruent to the femoral condyle (1b), and is integrally formed with the tibial plateau, **characterized in that** the entire femoral component (1), but at least the condylar surfaces (1a, 1b) thereof, consists of a firmly sintered ceramic, for articulation with the tibial component (2), and **in that** the tibial component (2) comprises a tibial plateau made of a firmly sintered ceramic.

2. Knee endoprosthesis comprising a femoral component (1), which has two condylar surfaces (1a, 1b), for anchoring to the distal femur, and comprising a tibial component (2) for anchoring to the proximal tibia, and comprising sliding surfaces between said two components (1, 2), the tibial plateau having a medial spherical bearing shell (7) on the upper face of said plateau that faces the femoral component (1), the medial spherical bearing shell (7) being an integral component of the tibial plateau and integrally formed therewith, and the articulation surface of the medial femoral condyle (1a) also being spherical and, together with the medial spherical bearing shell (7), constituting a congruent ball joint, and a bearing shell (8) being arranged laterally adjacent to the medial spherical bearing shell (7), which laterally arranged shell allows the rotation of the femoral component (1), is formed in the anterior-posterior direction from a plurality of radii, is not congruent to the femoral condyle (1b), and is an inlay which is movable relative to the tibial plateau, **characterized in that** the entire femoral component (1), but at least the condylar surfaces (1a, 1b) thereof, consists of a firmly sintered ceramic, for articulation with the tibial component (2), and **in that** the tibial component (2) comprises a tibial plateau made of a firmly sintered ceramic.

3. Knee endoprosthesis according to claim 1, **characterized in that** the tibial plateau has, on the lower face thereof which faces the proximal tibia, a bolt (6) for anchoring.

4. Knee endoprosthesis according to claim 2, **characterized in that** the tibial plateau has, on the lower face thereof which faces the proximal tibia, a bolt (6) for anchoring.

5. Knee endoprosthesis according to either claim 2 or claim 4, **characterized in that** the inlay consists of UHMWPE, PEEK, PAEK or composites, or of a firmly sintered ceramic or polyethylene.

6. Knee endoprosthesis according to any of claims 2, 4 or 5, **characterized in that** the inlay is movable in a groove (9) on the tibial plateau.

7. Knee endoprosthesis according to claim 6, **characterized in that** the groove (9) is formed in the anterior-posterior direction in a straight line or having a radius of curvature which determines the femoral rotation.

8. Knee endoprosthesis according to claim 7, **characterized in that** the femoral rotation is limited to at most 15 degrees of internal and external rotation.

9. Knee endoprosthesis according to either claim 1 or claim 3, **characterized in that** the bearing shell (8) has an elevation (10) in the anterior direction.

10. Knee endoprosthesis according to any of claims 2 and 4 to 8, **characterized in that** the inlay has an elevation (10) in the anterior direction.

11. Knee endoprosthesis according to any of claims 1 to 10, **characterized in that** the condylar surface (1b) articulates in the bearing shell (8) and, in addition to the flexion movement, can also have a translational movement in the anterior-posterior direction.

12. Tibial component of a knee endoprosthesis comprising a femoral component (1), which has two condylar surfaces (1a, 1b), for anchoring to the distal femur, and comprising a tibial component (2) for anchoring to the proximal tibia, and comprising sliding surfaces between these two components (1, 2) according to any of claims 1 to 11, wherein the tibial component (2) comprises a tibial plateau made of a firmly sintered ceramic, which plateau has a medial spherical bearing shell (7) on the upper face thereof which faces the femoral component (1), which bearing shell is an integral component of the tibial plateau and is integrally formed therewith and the articulation surface of the medial femoral condyle (1a) is also spherical and, together with the medial spherical bearing shell (7), constitutes a congruent ball joint, and a bearing shell (8) is arranged laterally adjacent to the medial spherical bearing shell (7), which laterally arranged shell allows the rotation of the femoral component (1), is formed in the anterior-posterior direction from a plurality of radii, is not congruent to the femoral condyle, and is either integrally formed with the tibial plateau or is an inlay which is movable relative to the tibial plateau.

## Revendications

1. Endoprothèse de genou comportant un composant fémoral (1) doté de deux surfaces condyliennes (1a, 1b) pour l'ancrage au fémur distal et un composant tibial (2) pour l'ancrage au tibia proximal et des surfaces de glissement entre ces deux composants (1, 2), le plateau tibial comportant, sur son côté supérieur orienté vers le composant fémoral (1), une coquille de coussinet sphérique médiale (7), la coquille de coussinet sphérique médiale (7) faisant partie intégrante du plateau tibial et étant réalisée en une seule pièce avec celui-ci, et la surface d'articulation du condyle fémoral médial (1a) étant également sphérique et constituant conjointement avec la coquille de coussinet sphérique médiale (7) une articulation sphérique congruente, et latéralement, à côté de la coquille de coussinet sphérique médiale (7), une coquille de coussinet non congruente (8) permettant la rotation du composant fémoral (1) est disposée, laquelle coquille de coussinet est formée d'une pluralité de rayons dans la direction antéro-postérieure, ne correspond pas au condyle fémoral (1b) et est réalisée en une seule pièce avec le plateau tibial, **caractérisée en ce que** le composant fémoral (1) entier, mais au moins ses surfaces condyliennes (1a, 1b), pour l'articulation avec le composant tibial (2), est constitué d'une céramique solidement frittée, **en ce que** le composant tibial (2) comprend un plateau tibial constitué d'une céramique solidement frittée.

2. Endoprothèse de genou comportant un composant fémoral (1) doté de deux surfaces condyliennes (1a, 1b) pour l'ancrage au fémur distal et un composant tibial (2) pour l'ancrage au tibia proximal et des surfaces de glissement entre ces deux composants (1, 2), le plateau tibial comportant, sur son côté supérieur orienté vers le composant fémoral (1), une coquille de coussinet sphérique médiale (7), la coquille de coussinet sphérique médiale (7) faisant partie intégrante du plateau tibial et étant réalisée en une seule pièce avec celui-ci, et la surface d'articulation du condyle fémoral médial (1a) étant également sphérique et constituant conjointement avec la coquille de coussinet sphérique médiale (7) une articulation sphérique congruente, et latéralement, à côté de la coquille de coussinet sphérique médiale (7), une coquille de coussinet non congruente (8) permettant la rotation du composant fémoral (1) est disposée, laquelle coquille de coussinet est formée d'une pluralité de rayons dans la direction antéro-postérieure, ne correspond pas au condyle fémoral (1b) et constitue un inlay mobile par rapport au plateau tibial, **caractérisée en ce que** le composant fémoral (1) entier, mais au moins ses surfaces condyliennes (1a, 1b), pour l'articulation avec le composant tibial (2), est constitué d'une céramique solidement frittée, **en ce que** le composant tibial (2) comprend un plateau tibial constitué d'une céramique solidement frittée.

3. Endoprothèse de genou selon la revendication 1, **caractérisée en ce que** le plateau tibial comporte un boulon (6) pour l'ancrage sur son côté inférieur orienté vers le tibia proximal.

4. Endoprothèse de genou selon la revendication 2, **caractérisée en ce que** le plateau tibial comporte un boulon (6) pour l'ancrage sur son côté inférieur orienté vers le tibia proximal.

5. Endoprothèse de genou selon la revendication 2 ou 4, **caractérisée en ce que** l'inlay est constitué de l'UHMWPE, du PEEK, du PAEK ou des composites ou d'une céramique ou d'un polyéthylène solidement fritté.

6. Endoprothèse de genou selon l'une des revendications 2, 4 ou 5, **caractérisée en ce que** l'inlay est mobile dans une rainure (9) sur le plateau tibial.

7. Endoprothèse de genou selon la revendication 6, **caractérisée en ce que** la rainure (9) est rectiligne dans la direction antéro-postérieure ou comporte un rayon de courbure déterminant la rotation fémorale.

8. Endoprothèse de genou selon la revendication 7, **caractérisée en ce que** la rotation fémorale est limitée à un maximum de 15 degrés de rotation interne et externe.

9. Endoprothèse de genou selon la revendication 1 ou 3, **caractérisée en ce que** la coquille de coussinet (8) présente une élévation (10) dans la direction antérieure.

10. Endoprothèse de genou selon l'une des revendications 2, 4 à 8, **caractérisée en ce que** l'inlay présente une élévation (10) dans la direction antérieure.

11. Endoprothèse de genou selon l'une des revendications 1 à 10, **caractérisée en ce que** la surface condylienne (1b) est articulée dans la coquille de coussinet (8) et peut présenter, en plus du mouvement de flexion, un mouvement de translation dans la direction antéro-postérieure.

12. Composant tibial d'une endoprothèse de genou comportant un composant fémoral (1) doté de deux surfaces condyliennes (1a, 1b) pour l'ancrage au fémur distal et un composant tibial (2) pour l'ancrage au tibia proximal et des surfaces de glissement entre ces deux composants (1, 2) selon l'une des revendications 1 à 11, le composant tibial (2) comprenant un plateau tibial constitué d'une céramique solidement frittée, lequel plateau tibial comporte sur son côté supérieur orienté vers le composant fémoral (1) une coquille de coussinet sphérique médiale (7) faisant partie intégrante du plateau tibial et réalisée en une seule pièce avec celui-ci, et la surface d'articulation du condyle fémoral médial (1a) étant également sphérique et constituant conjointement avec la coquille de coussinet sphérique médiale (7) une articulation sphérique congruente, et latéralement, à côté de la coquille de coussinet sphérique médiale (7) une coquille de coussinet non congruente (8) permettant la rotation du composant fémoral (1) est disposée, laquelle coquille de coussinet est formée d'une pluralité de rayons dans la direction antéro-postérieure, qui est soit réalisée en une seule pièce avec le plateau tibial, soit constitue un inlay mobile par rapport au plateau tibial.
